(19) Europäisches Patentamt
European Patent Office
Office européen des brevets

(11) Publication number: 0 011 400 A1

# (12) EUROPEAN PATENT APPLICATION

(21) Application number: 79302303.7

(22) Date of filing: 23.10.79

(51) Int. Cl.[3]: C 07 G 11/00
A 61 K 31/445, C 07 H 15/04

(30) Priority: 25.10.78 JP 130501/78

(43) Date of publication of application:
28.05.80 Bulletin 80/11

(84) Designated Contracting States:
DE FR GB

(71) Applicant: MEIJA SEIKA KAISHA LTD.
No. 4-16 Kyobashi 2-chome
Chuo-ku, Tokyo(JP)

(72) Inventor: Takemi, Koeda
No. 1-1 Goshoyama-cho
Nishi-ku Yokohama-shi, Kanagawa(JP)

(72) Inventor: Masayuki, Yokota
Sakonyama-danchi 9-3-504 No. 1186, Sakonyama
Asahi-ku Yokohama-shi, Kanagawa(JP)

(72) Inventor: Tetsutaro, Niizato
No. 2-3-10, Higashimonzen
Kawasakai-ku Kawasaki-shi, Kanagawa(JP)

(72) Inventor: Kazuko, Mizutani
No. 321 Hazawa-cho
Kanafawa-ku Yokohama-shi, Kanagawa(JP)

(72) Inventor: Takashi, Tsurucka
No. 1, Kawara-cho
Saiwai-ku Kawasaki-shi, Kanagawa(JP)

(72) Inventor: Shigeharu, Inoue
No. 16-2 Tsutsujigaoka
Midori-ku Yokohama-shi, Kanagawa(JP)

(74) Representative: Roberts, Peter William et al,
MARKS & CLERK Alpha Tower ATV Centre
Birmingham, B1 1TT(GB)

## (54) An aminoglycosidic antibiotic salt.

(57) An aminoglycosidic antibiotic salt comprises an aminoglycosidic antibiotic associated with D-glucaro-1,5 -lactam and has reduced toxicity, particularly to the auditory organs and/or kidneys.

EP 0 011 400 A1

Croydon Printing Company Ltd.

This invention relates to an aminoglycosidic antibiotic salt with reduced toxicity.

Aminoglycosidic antibiotics such as dibekacin (3', 4'-dideoxykanamycin B), tobramycin (3'-deoxykanamycin B), gentamicin, kanamycin A, bekanamycin (kanamycin B), amikacin and neomycin are widely used as antibacterial agents which are very effective for clinical treatment of the infections caused by Gram-positive, Gram-negative and acid-fast bacteria. However, it has been reported that these antibiotics, when administered to patients suffering from renal failure or other diseases, may induce disorders in the auditory organ or kidney, and as a result, such antibiotics have found only limited utility in clinical applications.

Thus, an agent for reducing the disorders in the auditory organ and kidney induced by aminoglycosidic antibiotics or an aminoglycosidic antibiotic with reduced toxicity has long been desired.

As a result of studies into the possibility of reducing the toxicity of aminoglycosidic antibiotics, it has now been found that a D-glucaro-1,5-lactam salt of an aminoglycosidic antibiotic produced by having D-glucaro-1,5-lactam act upon the aminoglycosidic antibiotic is less toxic than the aminoglycosidic antibiotic per se.

Accordingly, the invention resides in one aspect in an aminoglycosidic antibiotic salt comprising an aminoglycosidic antibiotic associated with D-glucaro-1, 5-lactam.

In a further aspect, the invention resides in an anti-microbial pharmaceutical composition including, as a main ingredient, a salt of aminoglycosidic antibiotic and

D-glucaro-1, 5-lactam in such an amount that the salt exhibits the intended antibiotic effect but has reduced toxicity as compared with the aminoglycosidic antibiotic per se.

D-glucaro-1,5-Lactam having the following formula:

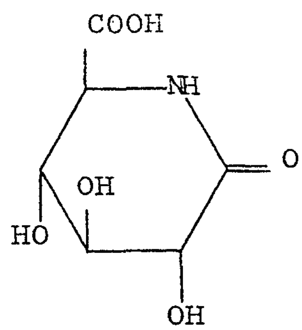

is already disclosed in Japanese Patent Publication 28,375/70. As its formula suggests, the D-glucaro-1, 5-lactam has a strong carboxylic acid group which renders it acidic and, therefore, it reacts readily with the amino group of an aminoglycosidic antibiotic in a solution and forms a salt. To be more specific, an addition of a D-glucaro-1,5-lactam aqueous or water-containing methanol solution to an aminoglycoside aqueous or water-containing methanol solution results in immediate formation of a salt thereof at room temperature. The salt may also be produced by having a calcium or barium salt of D-glucaro-1,5-lactam act on an aminoglycoside sulfate and removing the calcium or barium sulfate precipitated. The amount of the D-glucaro-1,5-lactam added varies with the number of the amino groups of the aminoglycoside and with the pH of the solution of the resulting salt, 2 to 5 moles of the lactam generally being used per mol of the aminoglycoside. The molar ratio of the lactam to glycoside should be relatively large for aminoglycosidic antibiotics having 5 to 6 amino groups such as dibekacin, tobramycin, gentamicin, bekanamycin and neomycin, whereas the ratio should be relatively

small for aminoglycosidic antibiotics having 4 amino groups such as kanamycin A and amikacin. An increased amount of D-glucaro-1,5-lactam is necessary for preparing a weak acidic salt within the pharmaceutically acceptable pH values and hence the molar ratio of the lactam to glycoside is not necessarily an integer. One reason for the variation in the molar ratio of the lactam to glycoside is that an aminoglycoside in the form of a free base often absorbs atmospheric carbon dioxide gas, and this results in a decrease in the amount of D-glucaro-1, 5-lactam to be added to the aminoglycoside.

The salt of the aminoglycoside and D-glucaro-1, 5-lactam (hereinafter referred to "the compound of this invention") is generally isolated in the form of a powder by concentrating a solution containing the compound of this invention to dryness, freeze-drying the solution, or forming a precipitate from the solution by addition of ethanol or acetone. The powder contains 0 to 8 mols of retained moisture depending on the manufacturing process or drying method.

In the accompanying drawings,

Figures 1 to 5 are the I.R. absorption spectra of the white powders produced in Examples 1 to 5, respectively, according to this invention.

Referring to the drawings, the physicochemical properties of the compounds produced in Examples 1 to 5, which are described in detail below, are as follows:-

(1) Salt of dibekacin and D-glucaro-1,5-lactam (the product of Example 1):

The salt is a white powder having a melting point of 132-137°C (with decomposition); is freely soluble in water, sparingly soluble in methanol, and very slightly soluble in ethanol, acetone, ethyl acetate and chloroform; and gives a positive result with the ninhydrin reaction. An aqueous solution of the salt has a pH of 7.4 to 7.6. The U.V. absorption shows no maximum. The salt has an I.R. absorption spectrum as indicated in Figure 1. Elemental analysis for $C_{18}H_{37}N_5O_8 \cdot (C_6H_9NO_6)_3 \cdot 4H_2O$:

| | | | |
|---|---|---|---|
| Calculated: | C: 39.42, | H: 6.62, | N: 10.21% |
| Found: | C: 39.19, | H: 6.68, | N: 10.20% |

Dibekacin content:

| | |
|---|---|
| Calculated: | 412 µg/mg |
| Found (by antibacterial assay): | 415 µg/mg |

(2) Salt of gentamicin and D-glucaro-1,5-lactam (the product of Example 2):

The salt is a white powder having a melting point of 146 - 148°C (with decomposition); is freely soluble in water, sparingly soluble in methanol, and very slightly soluble in ethanol, acetone, ethyl acetate and chloroform; and gives a positive result with the ninhydrin reaction. An aqueous solution of the salt has a pH of 6.7. The U.V. absorption shows no maximum. The salt has an I.R. absorption spectrum as indicated in Figure 2.

Elemental analysis for $C_{21}H_{43}N_5O_7 \cdot (C_6H_9NO_6)_4 \cdot 4H_2O$ (with gentamicin assumed gentamicin $C_{1a}$):

| | | | |
|---|---|---|---|
| Calculated: | C: 41.13, | H: 6.67, | N: 9.59% |
| Found: | C: 40.63, | H: 6.05, | N: 9.03% |

Gentamicin content:

Calculated: 340 µg/mg

Found (by antibacterial assay): 350 µg/mg

(3) Salt of tobramycin and D-glucaro-1,5-lactam (the product of Example 3):

The salt is a white powder having a melting point of 117-118°C (with decomposition); is freely soluble in water, sparingly soluble in methanol, and very slightly soluble in ethanol, acetone, ethyl acetate and chloroform, and gives a positive result with the ninhydrin reaction. An aqueous solution of the salt has a pH of 6.5. The U.V. absorption shows no maximum. The salt has an I.R. absorption spectrum as indicated in Figure 3.

Elementaly analysis for $C_{18}H_{37}N_5O_9 \cdot (C_6H_9NO_6)_4 \cdot 8H_2O$:

Calculated: C: 36.66, H: 6.53, N: 9.16%

Found: C: 35.71, H: 5.56, N: 8.14%

Tobramycin content:

Calculated: 339 µg/mg

Found: 350 µg/mg

(4) Salt of bekanamycin and D-glucaro-1,5-lactam (the product of Example 4):

The salt is a white powder having a melting point of 149-151°C (with decomposition); is freely soluble in water, and very slightly soluble in ethanol, acetone, ethyl acetate and chloroform; and gives a positive result with the ninhydrin reaction. The U.V. absorption shows no maximum. The salt has an I.R. absorption spectrum as indicated in Figure 4.

Elemental analysis for $C_{18}H_{37}N_5O_{10} \cdot (C_6H_9NO_6)_3 \cdot 2H_2O$:

Calculated: C: 39.56, H: 6.27, N: 10.25%

Found: C: 40.26, H: 5.94, N: 10.47%

Bekanamycin content:

Calculated: 422 µg/mg

Found: 400 µg/mg

(5) Salt of kanamycin A and D-glucaro -1,5-lactam (the product of Example 5):

The salt is a white powder having a melting point of 126-128°C (with decomposition): is freely soluble in water and very slightly soluble in ethanol, acetone, ethyl acetate and chloroform; and gives a positive result with the ninhydrin reaction. The U.V. absorption shows no maximum. The salt has an I.R. absorption spectrum as indicated in Fig. 5.

Elemental analysis for $C_{18}H_{36}N_4O_{11} \cdot (C_6H_9NO_6)_{2.5} \cdot 4H_2O$:

Calculated: C: 38.32, H: 6.48, N: 8.80%

Found C: 38.89, H: 6.26; N: 8.69%

Kanamycin A content:

Calculated: 468 µg/mg

Found: 420 µg/mg

An antimicrobial pharmaceutical composition that contains the compound of this invention as the main ingredient is applicable to the treatment of humans and animals such as monkeys, horses, cows, dogs, cats, domestic fowl, pigs and sheep, and is administered ................

in the same manner as known aminoglycosidic antibiotic, for instance, by intramuscular, intravenous or subcutaneous injection, oral adminis ration, rectal infusion or lacal administration.

An injection may be a dry product which can be rendered liquid by being dissolved in water or any other suitable vehicle prior to use. A liquid preparation such as an aqueous solution, oily suspension or emulsion may also be used as an injection. Suitable aqueous vehicles are sterile distilled water and physiological saline solution, and suitable nonaqueous vehicles are a vegetable oil such as sesame oil, an animal oil such as squalene, or propylene glycol. A preferred suspension is a W/O suspension. The liquid preparation may contain a suspending agent such as methyl cellulose, an emulsifying agent such as lecithin, a preservative such as methyl-p-hydroxybenzoate, or a conventional stabilizer.

For rectal infusion, the antimicrobial composition preferably takes a form of a suppository which may use cocoa butter or other glycerides as a base.

For oral administration, a tablet, capsule, powder, dry syrup or a liquid preparation such as syrup is conveniently used. The tablet or capsule may contain a binder such as gelatin, a filler such as calcium phosphate, a lubricant such as talc, a disintegrator such as potato starch, or a wetting agent such as sodium lauryl sulfate in the conventional manner. For local administration, these liquid preparations or ointment is used conveniently.

Since D-glucaro-1,5-lactam has no effect on the

antibacterial activity of the compound of this invention, the amounts of the conventional aminoglycosides as calculated for clinical use in terms of antibacterial potency can serve a direct substitute for the dosage of the compound of this invention. A high dose of the compound of this invention can be administered without inducing any side-effect to the patients who require a high dose of aminoglycoside. For example, the salt of dibekacin and D-glucaro-1,5-lactam is administered to a human adult at a dose of 50 to 200 mg potency (as a free base of dibelacin) daily.

This invention is now described in greater detail by reference to the following examples which are given here for illustrative purposes only and are by no means intended to limit the scope of this invention.

Example 1

9.04 g of dibekacin (as a free base) was dissolved in 50 ml of distilled water. To the resulting solution was added 50 ml of distilled water having dissolved therein 11.46 g of D-glucaro-1,5-lactam (as a free acid). The resulting solution was concentrated to dryness and, further, dried by phosphorous pentoxide under vacuum to obtain 19.8 g of a white powder of the salt of dibekacin and D-glucaro-1,5-lactam (associated in a molar ratio of 1:3). Melting point: 132 - 133°C (with decomposition)

Example 2

6.71 g of gentamicin (as a free base) was dissolved in 20 ml of distilled water. To the resulting solution was added 40 ml of distilled water having dissolved therein 14.33 g

of D-glucaro-1,5-lactam (as a free acid). Addition of 500 ml of acetone to the mixture resulted in the formation of a white precipitate, which was filtered off and vacuum dried to obtain 20.46 g of a white powder of the salt of gentamicin and D-glucaro-1,5-lactam (associated in a molar ratio of 1:5). Melting point: 146 - 148°C (with decomposition).

Example 3

4.67 g of tobramycin (as a free base) was dissolved in 15 ml of distilled water. To the resulting solution was added 20 ml of distilled water having dissolved therein 7.64 g of D-glucaro-1,5-lactam (as a free acid). The resulting solution was concentrated and freeze-dried to obtain 12,83 g of a white powder of the salt of tobramycin and D-glucaro-1,5-lactam (associated in a molar ratio of 1:4). Melting point: 117-118°C (with decomposition)

Example 4

6.3 g of bekanamycin sulfate was dissolved in 40 ml of distilled water. To the resulting solution was added 50 ml of distilled water having dissolved therein 6.33 g of calcium salt of D-glucaro-1,5-lactam, upon which the precipitate of calcium sulfate formed. The precipitate was filtered off and the filtrate was concentrated to dryness, washed with ethanol and vacuum dried to obtain 9.62 g of a white powder of the salt of bekanamycin and D-glucaro-1,5-lactam (associated in a molar ratio of 1:3). Melting point: 149 - 151°C (with decomposition)

Example 5

9.68 g of kanamycin A (as a free base) was dissolved in

40 ml of distilled water. To the resulting solution was added 60 ml of an aqueous solution containing 11.46 g of D-glucaro-1,5-lactam (as a free acid). The resulting solution was concentrated to dryness, washed with ethanol and vacuum dried to obtain 19.67 g of a white powder of the salt of kanamycin A and D-glucaro-1,5-lactam (associated in a molar ratio of 1:3). Melting point: 126 - 128°C (with decomposition)

Example 6

Male Hartley guinea pigs weighing 300 g on average were used as the test animals in three groups (the first two consisting of 9 guinea pigs and the third onsisting of 6 guinea pigs) which were subjected to intramuscular injection of the following substances for 14 consecutive days. The amount of the test compounds administered was calculated on the basis of the free base (i.e., free gentamicin). The first group was injected with 100 ml (as a free base)/kg/day of a solution of the salt of gentamicin and D-glucaro-1,5-lactam in water for injection. The second group was injected with an equal dose of a solution of gentamicin sulfate in water for injection. The third group was a control which was injected with distilled water. After injection with the final dose, each group was subjected to a pure tone having a frequency of 20,000 Hz to determine the pinna reflex of the subject guinea pigs. Twenty-four hours after the determination, the levels of blood urea nitrogen (BUN) and creatine (CRE) in sera were measured. The results are set forth in Tables 1 and 2 below.

Table 1

| Test Compound | Number died/ Number tested | Pinna Reflex | | |
|---|---|---|---|---|
| | | Normal | Dull | Lost |
| Control | 0/6 | 6/6 | | |
| Glucarolactam salt of gentamicin | 2/9 | 6/7 | - | 1/7 |
| Gentamicin sulfate | 7/9 | - | 2/5 | 3/5 |

Table 2

| Test Compound | Number tested | BUN (mg/dℓ) | CRE |
|---|---|---|---|
| Control | 6 | 22.6±1.21 | 0.63±0.08 |
| Glucarolactam salt of gentamicin | 7 | 64.6±43.97 | 1.20±0.51 |
| Gentamicin sulfate | 2 | 92.3±50.42 | 1.80±0.57 |

Example 7

Male Hartley guinea pigs weighing 300 g on average were used as the test animals in three groups each consisting of 6 guinea pigs and subjected to intramuscular injection of the following substances for 21 consecutive days. The first group was injected with 180 mg (as a free base)/kg/day of a solution of the salt of dibekacin and D-glucaro-1,5-lactam in water for injection. The second group was injected with an equal dose of a solution of dibekacin sulfate in water for injection. The third group was a control which was injected with distilled water. During the intramuscular injection, the pinna reflex of each subject guinea pig to a pure tone having a frequency of 20,000 Hz was determined. Twenty-four hours after the injection with the final dose, the level of BUN in sera was

measured. The results are set forth in Table 3 below.

Table 3

| Test Compound | Number died/ Number tested | Number of guinea pigs which lost pinna reflex | | | | BUN (mg/dℓ) |
|---|---|---|---|---|---|---|
| | | 10days | 14days | 17days | 21days | |
| Control | 0/6 | 0/6 | 0/6 | 0/6 | 0/6 | 30.1±2.34 |
| Glucarolactam salt of dibekacin | 0/6 | 0/6 | 0/6 | 0/6 | 3/6 | 35.7±13.3 |
| Dibekacin sulfate | 1/6 | 0/6 | 3/6 | 3/6 | 5/6* | 41.8±26.9 |

* including dead cases

Example 8

Male Hartley guinea pigs weighing 300 g on average were used as the test animals in three groups (the first two consisting of 9 guinea pigs and the third consisting of 6 guinea pigs) which were subjected to intramuscular injection with 0.5 ml of the following substances daily for 16 consecutive days. The first group was injected with a solution of the salt of tobramycin and D-glucaro-1,5-lactam in water for injection in a dose of 120 mg (as a free base)/kg/day. The second group was injected with a solution of tobramycin sulfate in water for injection in an equal dose. The third group was a control which was injected with distilled water. After injection with the final dose, the pinna reflex of each subject guinea pig to a pure tone having a frequency of 15,000 Hz was determined. In the same manner as above, two other groups (6 each) of male Hartley guinea pigs weighing 300 g on average were subjected to intramuscular injection of the following substances for 14 consecutive days. The fourth group was

injected with a solution of the salt of bekanamycin and D-glucaro-1,5-lactam in water for injection in a dose of 250 mg (as a free base)/kg/day, whereas the fifth group was injected with a solution of bekanamycin sulfate in water for injection in an equal dose. Both group were then subjected to determination of their pinna reflex to a 15,000 Hz pure tone. The results are set forth in Table 4 below.

Table 4

| Test Compound | Number died/ Number tested | Pinna Reflex | | |
|---|---|---|---|---|
| | | Normal | Dull | Lost |
| Control | 0/6 | 6/6 | - | - |
| Glucarolactam salt of tobramycin | 0/9 | 9/9 | - | - |
| Tobramycin sulfate | 0/9 | 7/9 | 2/9 | - |
| Glucarolactam salt of bekanamycin | 0/6 | - | 3/6 | 3/6 |
| Bcanamycin sulfate | 2/6 | - | 1/4 | 3/4* |

* including dead cases

## Example 9

Male Hartley guinea pigs weighing from 300 to 500 g were used as the test animals in four groups each consisting of 6 guinea pigs which were subjected to intramuscular injection with 0.5 ml of the following substances for 14 consecutive days. The first group was injected with a solution of the salt of kanamycin A and D-glucaro-1,5-lactam in water for injection in a dose of 400 mg (as a free base)/kg/day. The second group was injected with a solution of kanamycin A sulfate in water for injection in an equal dose. The third group was injected

with a solution of a mixture of kanamycin A sulfate and potassium salt of D-glucaro-1,5-lactam (in a molar ratio of 1:2:5) in water for injection in an eaual dose. The fourth group was a control which was injected with distilled water. The results of determination of the pinna reflex (to a 15,000 Hz pure tone) after injection with the final dose and those of measurement of the BUN level in sera 24 hours after injection with the final dose are set forth in Table 5 below.

Table 5

| Test Compound | Number died/ Number tested | Pinna Reflex | | | BUN (mg/dℓ) |
|---|---|---|---|---|---|
| | | Normal | Dull | Lost | |
| Control | 0/6 | | | | 22±1.2 |
| Glucarolactam salt of kanamycin | 0/6 | - | 4/6 | 2/6 | 29±5.4 |
| Kanamycin sulfate | 0/6 | - | - | 6/6 | 37±9.6 |
| Mixture of Kanamycin sulfate and potassium salt of glucarolactam | 0/6 | - | - | 6/6 | 29±3.7 |

## Example 10

Male ICR-JCL mice weighing 20.4 g on average were used as the test animals in three groups each consisting of 10 mice, which were inoculated intraperitoneally with a solution containing 7.1 x $10^4$ cells of Pseudomonas aeruginosa E-2/mouse/ 0.5 ml (containing 2.5% mucin) and immediately thereafter, subjected to intramuscular injection in the thigh with 0.2 ml of the following substances. The first group was injected with a solution of the salt of dibekacin and D-glucaro-1,5-lactam in sterile physiological saline solution in doses of 1 mg (as a free base)/

mouse and 0.0625 mg (as a free base)/mouse. The second group was injected with a solutio of dibekacin sulfate in sterile physiological saline solution in the above indicated doses. The third group was a control which was injected with distilled water. The percentage survival of each group was checked one week after the injection. The results are set forth in Table 6 below.

Table 6

| Test Compound | Percentage Survival (%) Dosage 1 mg | 0.25 mg | 0.0625 mg | $ED_{50}$ (mg/kg) |
|---|---|---|---|---|
| Control | 0 | 0 | 0 | |
| Glucarolactam salt of dibekacin | 100 | 20 | 0 | 19.1* |
| Dibekacin sulfate | 100 | 30 | 0 | 15.7* |

* The two values as calculated by Ritchfield-Wilcoxon technique were not significantly different.

Example 11

Male Hartley guinea pigs weighing 300 g on average were used as the test animals in three groups each consisting of 6 guinea pigs which were subjected to intramuscular injection of the following substances for 21 consecutive days. The first group was injected with a solution of the salt of gentamicin and D-glucaro-1,5-lactam in water for injection in a dose of 80 mg (as a free base)/kg/day. The second group was injected with a solution of gentamicin sulfate in water for injection in an equal dose. The third group was a control which was injected with distilled water. The results of determination of the pinna reflex (to a 20,000 Hz pure tone) after injection with the final dose are set forth in Table 7 below.

Table 7

| Test Compound | Pinna Reflex | | |
|---|---|---|---|
| | Normal | Dull | Lost |
| Control | 6/6 | 0/6 | 0/6 |
| Glucarolactam salt of gentamicin | 3/6 | 2/6 | 1/6 |
| Gentamicin sulfate | 0/6 | 1/6 | 5/6* |

* including one guinea pig dead on the 21th day

CLAIMS

1. An aminoglycosidic antibiotic salt comprising an aminoglycosidic antibiotic associated with D-glucaro-1,5-lactam.

2. A salt as claimed in claim 1 wherein said aminoglycosidic antibiotic is dibekacin.

3. A salt as claimed in claim 1 wherein said aminoglycosidic antibiotic is tobramycin.

4. A salt as claimed in claim 1 wherein said aminoglycosidic antibiotic is gentamicin.

5. A salt as claimed in claim 1 wherein said aminoglycosidic antibiotic is kanamycin A.

6. A salt as claimed in claim 1 wherein said aminoglycosidic antibiotic is kanamycin B.

7. A salt as claimed in any preceding claim wherein the aminoglycosidic antibiotic is associated with D-glucaro-1,5-lactam in a molar ratio of from 1:2 to 1:5

8. A salt as claimed in any preceding claim and having reduced toxicity to auditory organs and/or kidneys.

9. An antimicrobial pharmaceutical composition including, as a main ingredient, a salt of aminoglycosidic antibiotic and D-glucaro-1,5-lactam in such an amount that the salt exhibits the intended antibiotic effect but has reduced toxicity as compared with the aminoglycosidic antibiotic per se.

10. A pharmaceutical composition as claimed in claim 9 and having reduced toxicity to auditory organs and/or kidneys.

0011400

1/5

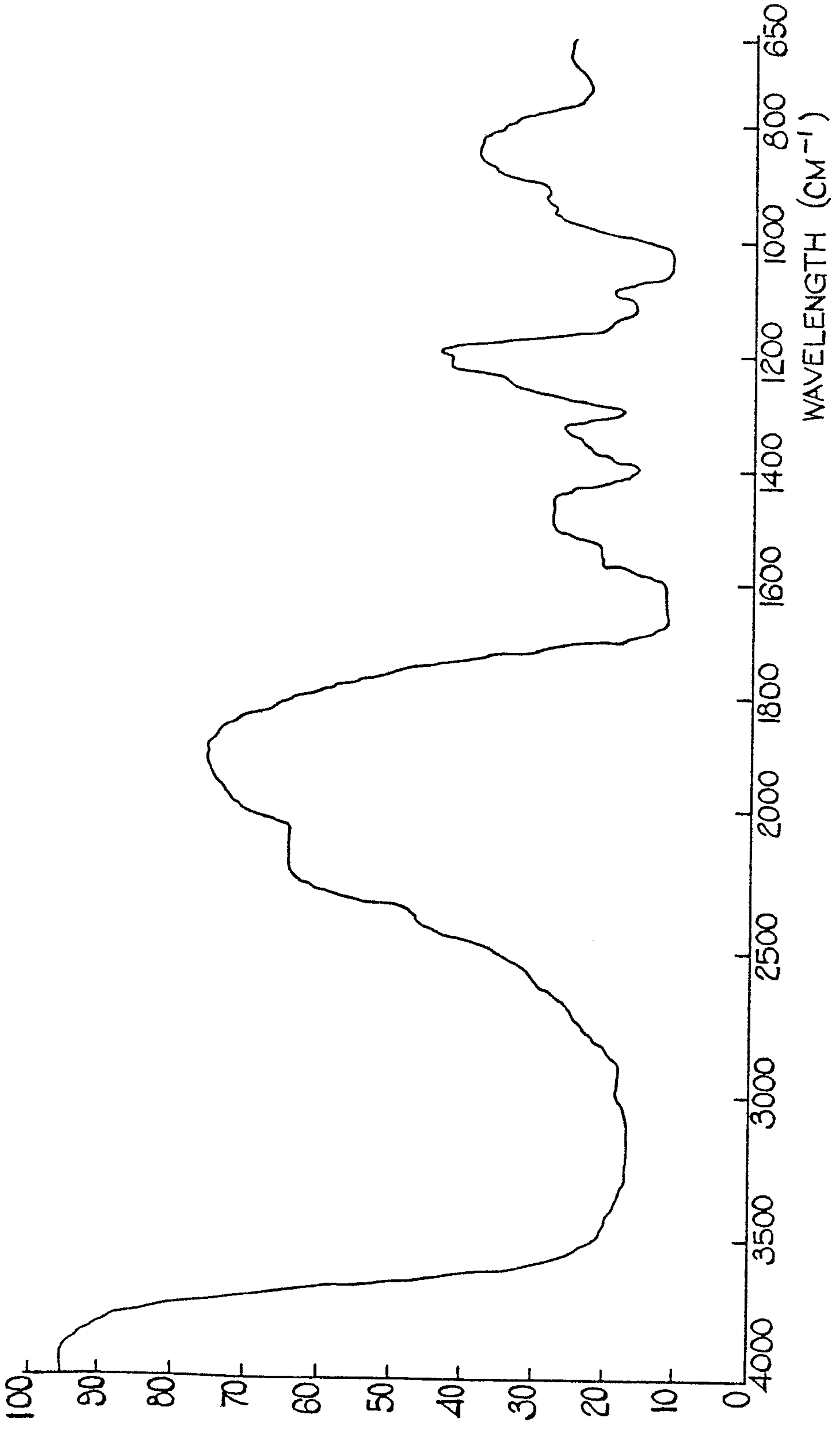


FIG.1.

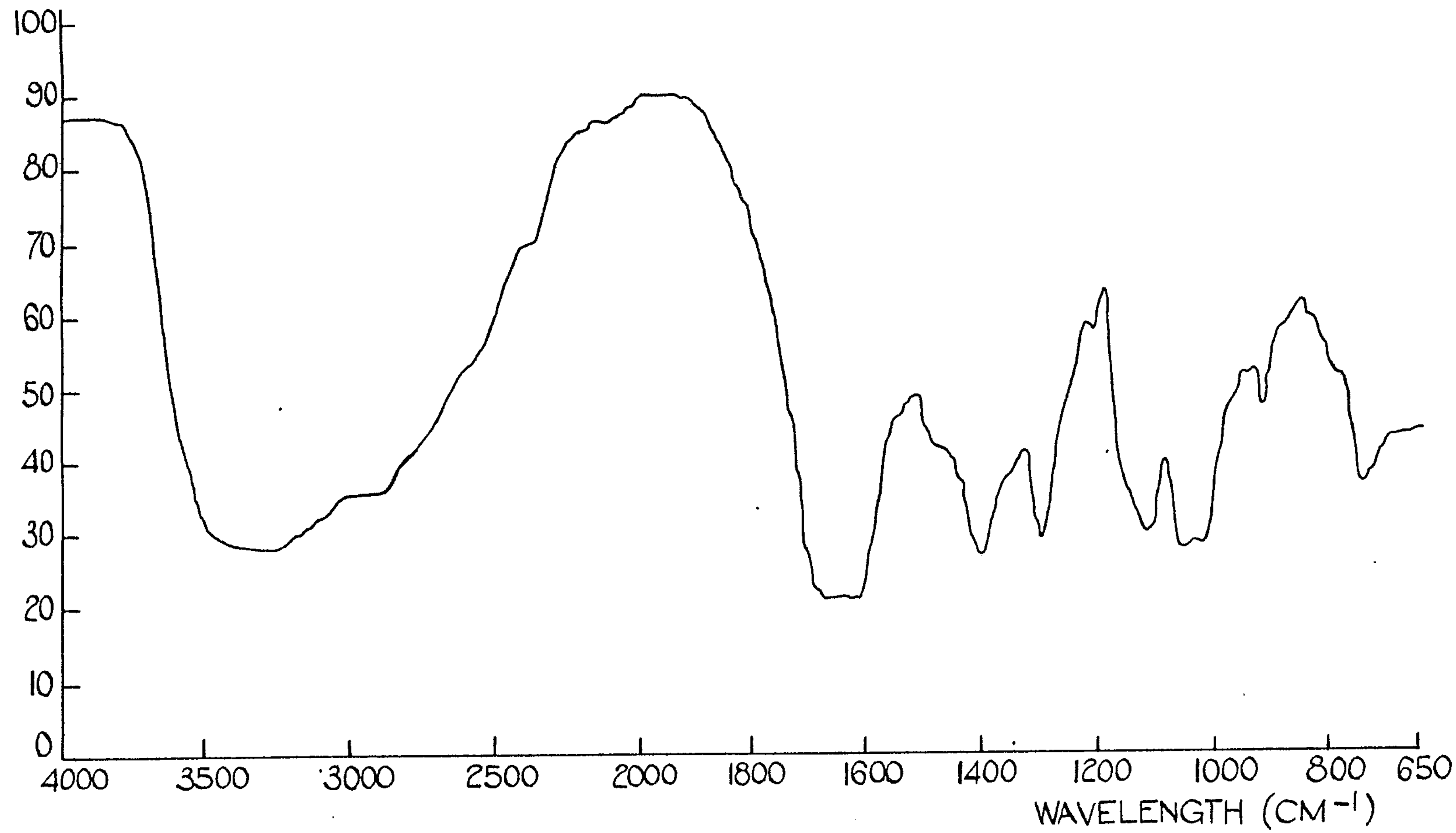
100
90
80
70
60
50
40
30
20
10
0
4000
3500
3000
2500
2000
1800
1600
1400
1200
1000
800
650
WAVELENGTH (CM⁻¹)

FIG.2.

0011400



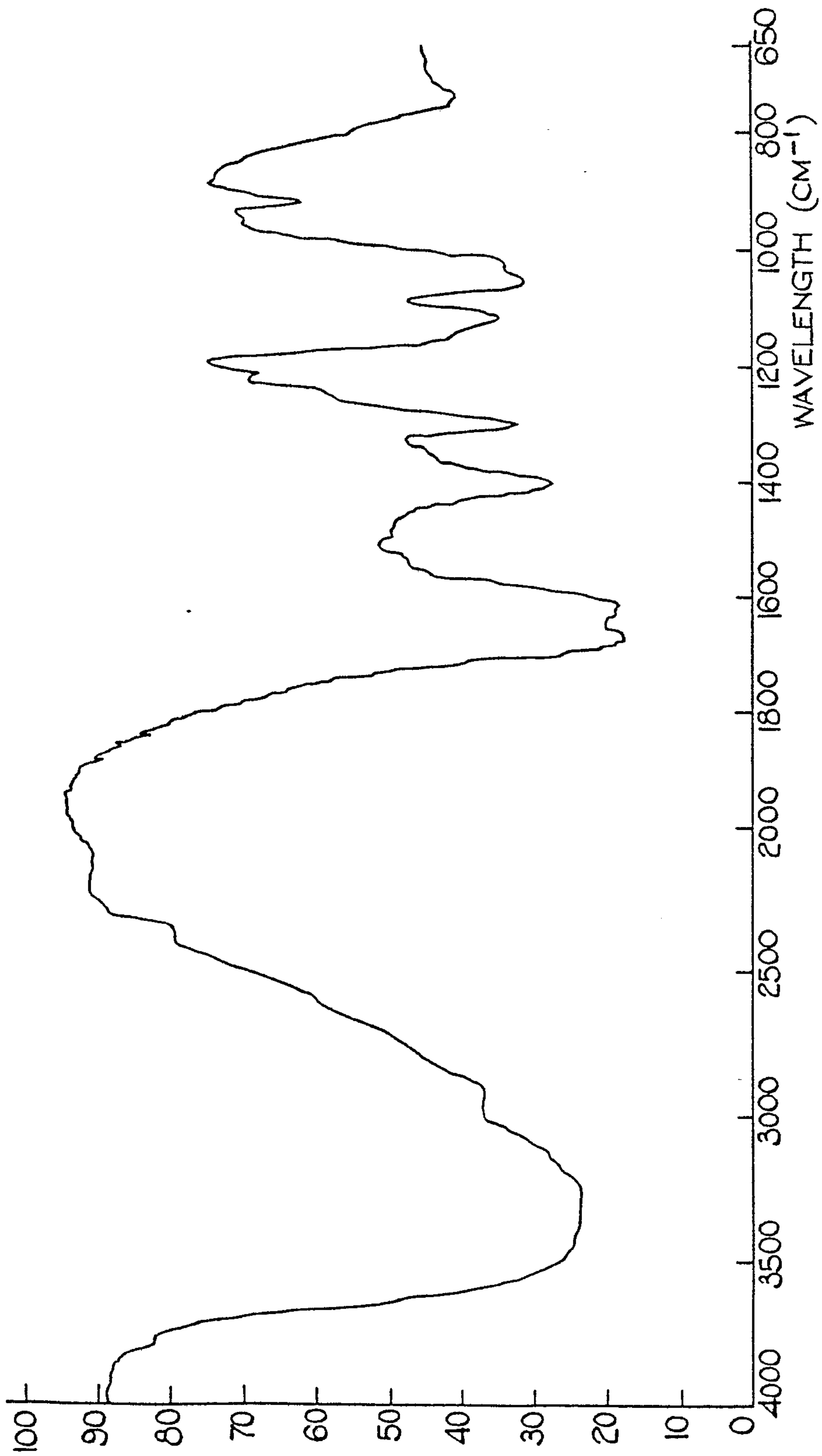


FIG.3.

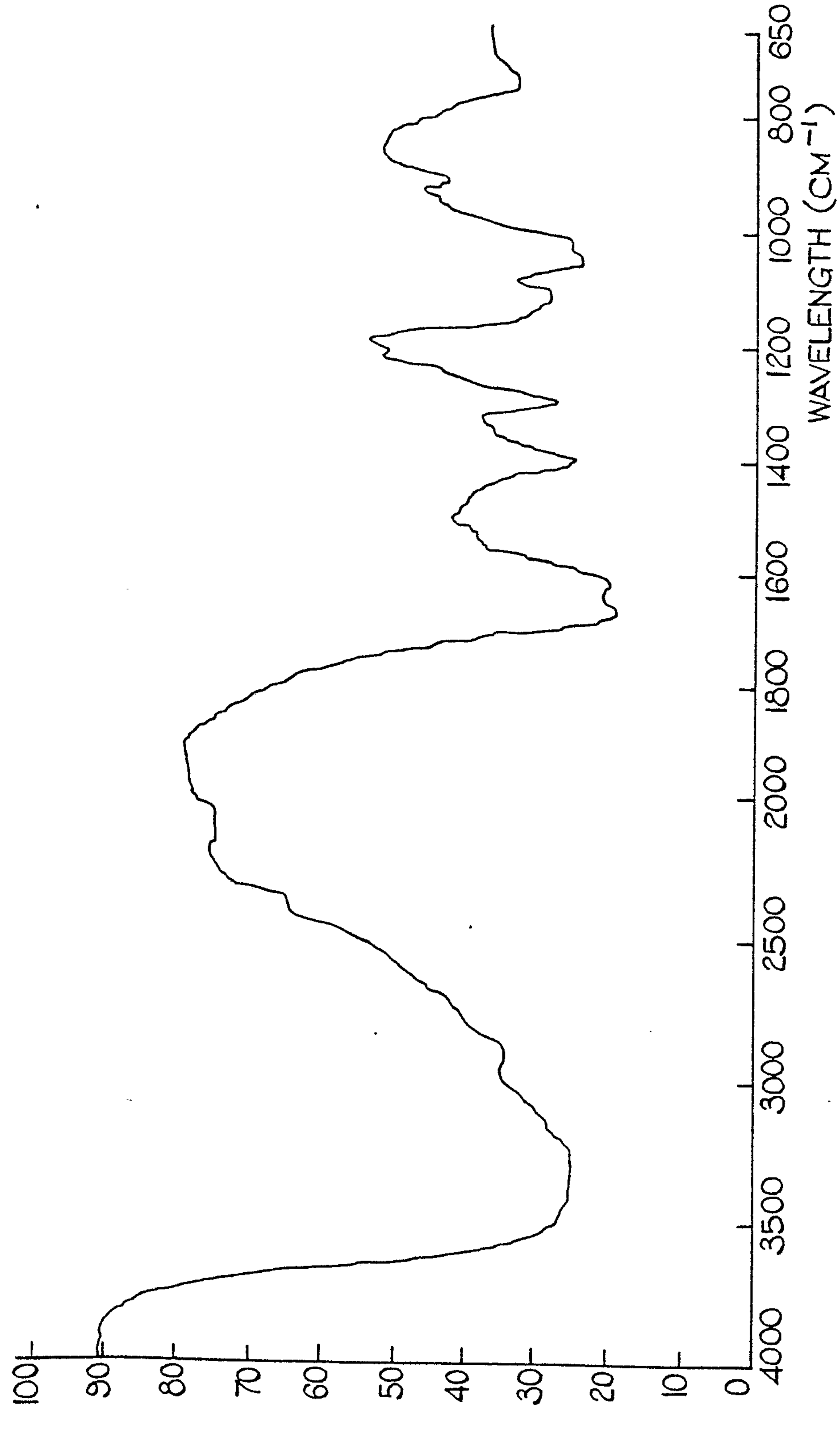
100
90
80
70
60
50
40
30
20
10
0
4000
3500
3000
2500
2000
1800
1600
1400
1200
1000
800
650
WAVELENGTH (CM$^{-1}$)

FIG. 4.

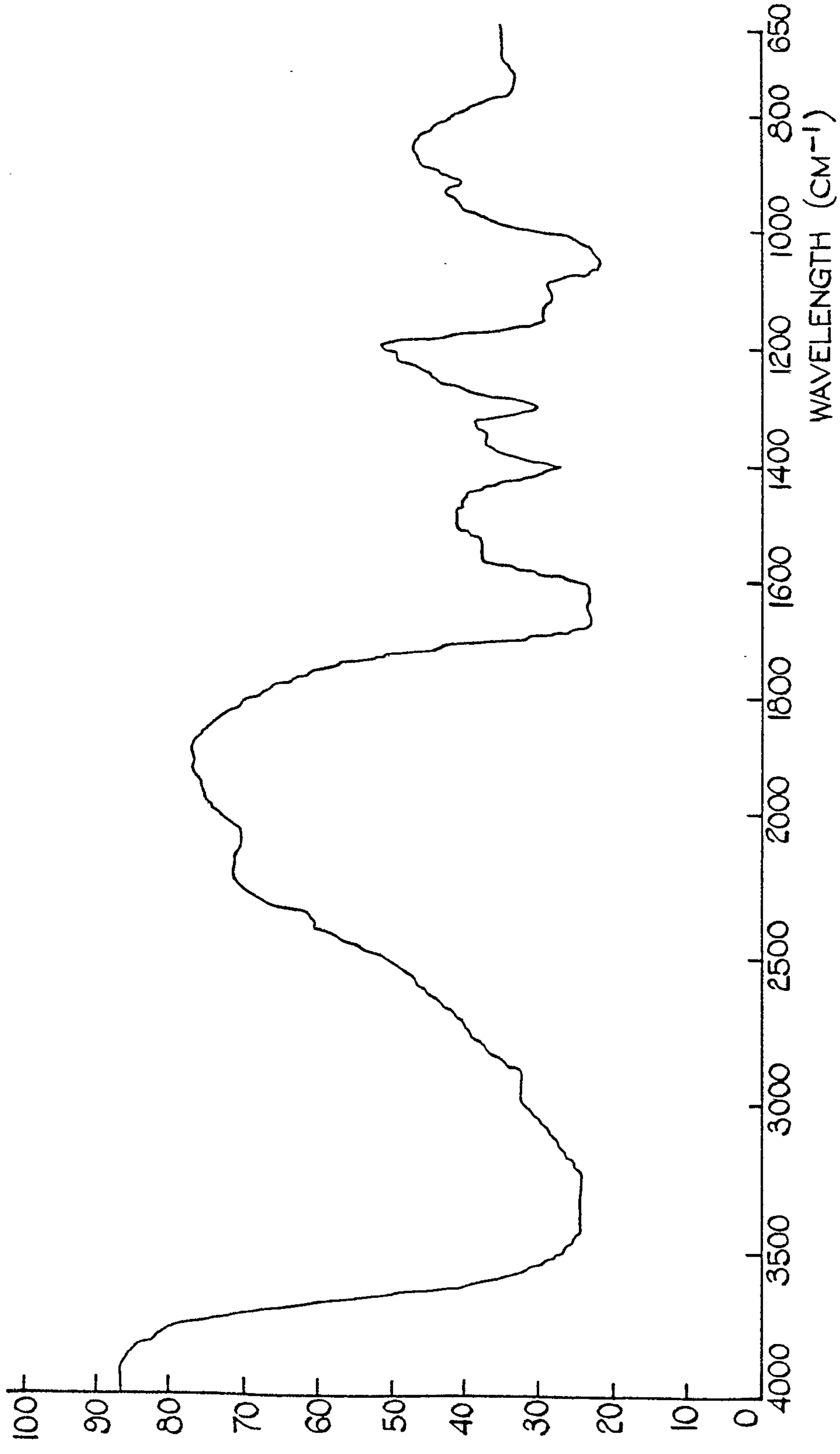
100
90
80
70
60
50
40
30
20
10
0
4000
3500
3000
2500
2000
1800
1600
1400
1200
1000
800
650
WAVELENGTH (CM-1)

FIG.5.

0011400

Application number

European Patent Office

# EUROPEAN SEARCH REPORT

EP 79 302 303.7

## DOCUMENTS CONSIDERED TO BE RELEVANT

| Category | Citation of document with indication, where appropriate, of relevant passages | Relevant to claim |
|---|---|---|
| X | US - A - 4 122 171 (T.NIIZATO et al.)<br>* whole document *<br>-- | 1,4-8 |
| | Chemical Abstracts vol. 85, no.21, 1976 Columbus, Ohio, USA<br>T. NIIZATO et al. "Protective effect of D-glucaro-δ-lactam against aminoglycoside-induced nephrotoxicity in rats"<br>page 30, column 1, abstract no. 153897c<br>& J. Antibiot. vol. 29, no. 8, 1976 pages 833 to 840<br>---- | 1,2,<br>5,6 |

**CLASSIFICATION OF THE APPLICATION (Int. Cl.³)**

C 07 G 11/00
A 61 K 31/445
C 07 H 15/04

**TECHNICAL FIELDS SEARCHED (Int.Cl.³)**

A 61 K 31/445
A 61 K 35/66
A 61 K 35/70
C 07 G 11/00
C 07 H 15/04
C 07 H 17/02
C 07 H 19/04

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 30-01-1980 | KNAACK |

EPO Form 1503.1 06.78